# EUROPÄISCHE PATENTSCHRIFT

(11) **EP 0 900 593 B1**
(45) Veröffentlichungstag und Bekanntmachung des Hinweises auf die Patenterteilung: **09.04.2003**
(21) Anmeldenummer: 98113650.0
(22) Anmeldetag: 22.07.1998
(51) Int. Cl.: B01J 31/10

(54) **Verwendung von hochtemperaturbeständigen sulfonierten aromatischen Polyetherketon-Kationenaustauschern als Katalysatoren**
Use of high-temperature resistant sulfonated aromatic polyetherketone cation exchangers as catalysts
Utilisation d'échangeurs cationiques à base de polyéthercétone aromatique sulfonée en tant que catalyseur

(30) Priorität: 29.07.1997 DE 19732578
(43) Veröffentlichungstag der Anmeldung: 10.03.1999
(73) Patentinhaber: Siemens Axiva GmbH & Co. KG, 65926 Frankfurt am Main (DE)
(72) Erfinder: Sievers, Werner, Dr., 65929 Frankfurt (DE); Daiminger, Ulrich, Dr., 65189 Wiesbaden (DE); Bönsel, Harald, Dr., 65529 Waldems (DE); Bauer, Wolfgang, 65795 Hattersheim (DE); Semel, Joachim, Dr., 61462 Königstein (DE)
(74) Vertreter: Berg, Peter, Dipl.-Ing.

(56) Entgegenhaltungen:
- EP-A- 0 574 791
- WO-A-96/13073
- WO-A-97/19480
- DE-A- 19 527 435
- US-A- 5 371 253
- Y. Z. YUAN ET AL.: "Sulfonated polyphenylene-sulfide as ligands in Rh-complexes catalyzed hydroformylation" CHINESE CHEMICAL LETTERS, Bd. 4, Nr. 2, 1993, Seiten 163-166, XP002101373

## Beschreibung

Auf der Basis von aromatischen Polyetherketonen wurden gemäß den Patentschriften EP 0 575 807 A1, EP 0 574 791 A2, DE 195 10 026 A1 und DE 195 27 435.0 auf die hier ausdrücklich Bezug genommen wird, saure Kationenaustauscher hergestellt, die sich durch enorm hohe Temperaturstabilität auszeichnen. Als Dauerbelastung sind Temperaturen bis 240°C zulässig, bei kurzzeitiger Belastung sind Spitzentemperaturen bis 280°C möglich. Die extreme Temperaturstabilität resultiert aus der Verwendung der oben beschriebenen Ausgangsmaterialien und des speziellen Herstellungsverfahrens. Auf dem Markt angebotene, saure Kationenaustauscherharze werden hauptsächlich auf der Basis von vernetzten Styrol-Divinylbenzol oder Acryl hergestellt und weisen eine maximale Arbeitstemperatur von 150°C auf. Sie werden üblicherweise als protonenleitende Membranen verwendet, beispielsweise in Brennstoffzellen.
Weiter sind aus der WO 97/19480 A1 und der EP 0 574 791 A2 Katalysatoren bekannt, die aromatische Polyetherketone enthalten, jedoch nicht als katalytisch aktive Materialien.

Es wurde nun überraschend gefunden, daß sich sulfonierte aromatische Polyetherketon-Kationenaustauscher auf einem anderen als dem genannten technischen Gebiet einsetzen lassen, und zwar als Katalysatoren bei heterogen säurekatalysierten Reaktionen. Dies ist um so überraschender, als es bislang keine sauren lonenaustauscher auf Basis von organischen Monomeren gibt.

Gegenstand der Erfindung ist daher die Verwendung von sulfonierten aromatischen Polyetherketon-Kationenaustauschern als Katalysatoren.

Bei heterogen katalysierten Reaktionen kann, im Gegensatz zu homogen katalysierten Reaktionen, der Ort der Reaktion genau definiert werden und darüber hinaus entfallen die Abtrennung, Aufarbeitung und Rezyklierung des Katalysators. Aufgrund physikalisch chemischer oder wirtschaftlicher Gründe ist bei zahlreichen heterogen katalysierten Reaktionen weiterhin eine hohe Arbeitstemperatur grundsätzlich nötig oder zumindest sehr vorteilhaft. Es ist allgemein bekannt, daß durch hohe Temperaturen chemische Reaktionen beschleunigt oder überhaupt erst möglich gemacht werden. Typische Beispiele für solche Reaktionen sind Veresterungen / Esterspaltung, Veretherungen / Etherspaltung, Acetalisierung / Acetalspaltung, Eliminierungen / Hydratisierung und Alkylierungen. In allen diesen säurekatalysierten Verfahren können die erwähnten, hochtemperaturbeständigen Ionenaustauscher vorteilhaft eingesetzt werden. Dies ist unabhängig vom Apparatetyp, der als Kesselreaktor oder Rohrreaktor etc. ausgeführt sein kann.
Dabei ergibt sich als weiterer überraschender Vorteil von sauren Kationentauschern auf Basis von aromatischen Polyetherketonen, daß durch die mögliche Einstellung des Grades an lonentauscheräquivalenten selektiv Reaktionen bei hohen Temperaturen beschleunigt werden können: so wird in Carbonsäure / Alkohol - Gemischen bei hohen Temperaturen bei geringen Sulfonierungsgraden bevorzugt die Veresterung katalysiert, und bei höheren Sulfonierungsgraden bevorzugt die Veretherung der vorhandenen Alkohole. Weiterhin können die hochtemperaturbeständigen Kationenaustauscher in Verfahren wie der Reaktivdestillation eingesetzt werden, in denen gleichzeitig mit der Reaktion eine Stofftrennung der einzelnen Produkte erfolgt. Auch hier zeigen sich deutliche Vorteile von hochtemperaturbeständigen Kationentauschern, da gerade bei der überlagerten rektifikativen Stofftrennung oft Temperaturen von über 150°C benötigt werden.

Die Herstellung der Kationenaustauscher zur erfindungsgemäßen Verwendung kann folgendermaßen erfolgen: Das aromatische Polymer wird in Schwefelsäure von 94-97 Gew.-% gelöst und die Lösung mit einem sulfonierenden Agens, z.B. Oleum, versetzt, bis die Schwefelsäurekonzentration 98-99.9 Gew.-% beträgt. Ist der gewünschte Sulfonierungsgrad erreicht, wird der Reaktionsansatz aufgearbeitet.
Die Materialien fallen bis zu lonenaustauscheräquivalenten von 1,0 mMol SO₃H/g als in NMP (N-Methyl-2-pyrrolidon) unlösliche Pulver an. Ab lonenaustauscheräquivalenten von 1,0 mMol SO₃H/g werden die Polymere in NMP oder DMSO (Dimethylsulfoxid) löslich. Wird der Sulfonierungsgrad weiter erhöht, steigt die Polarität des Polymers, und ab einem lonenaustauscheräquivalent von 1,8 mMol SO₃H/g wird das Material wasserlöslich.
Diese hochtemperaturbeständigen lonenaustauscher zeigen als Derivate von Hochleistungspolymeren erstaunlich hohe chemische und thermische Beständigkeit. Nach DSC- und TGA-Untersuchungen kann bei Heizraten von 10°C/min ein thermisches Desulfonieren erst oberhalb von 300°C beobachtet werden.

Nachstehendes Beispiel dient der weiteren Erläuterung der Erfindung. Eine Beschränkung der Erfindung in irgendeiner Weise ist dadurch nicht beabsichtigt.

Am Beispiel der Veresterung von Palmitinsäure zu Palmitinsäureoktylester, soll der vorteilhafte Einsatz der neuen, hochtemperaturbeständigen Kationenaustauscher im Vergleich zu auf dem Markt erhältlichen Produkten dargestellt werden. Die sauer katalysierte Reaktion von Palmitinsäure (Siedetemperatur 350°C) und Oktanol (Siedetemperatur 195 °C) zu Palmitinsäureoktylester und Wasser wurde bei 200°C in einem gerührten Kessel bei Normaldruck durchgeführt. Im beheizbaren Rührbehälter wurden 265 Gramm Oktanol und 522 Gramm Palmitinsäure vorgelegt. Nach Aufheizen des Behälters auf 190-195°C mittels eines Ölthermostaten wurden 100 Gramm des jeweiligen lonenaustauschers in Pulver- bzw. Granulatform dazugegeben. Die über Kopf gehenden Brüden, in diesem Fall der als Produkt entstehende Wasserdampf, werden in einem Liebigkühler kondensiert und in einer auf einer Waage stehenden Vorlage aufgefangen.
Als Katalysatoren wurden eingesetzt: ein hochtemperaturbeständiges Kationenaustauscherharz auf PEEKK-Basis (Polyetheretherketonketon), ein hochtemperaturbeständiges Kationenaustauscherharz auf PEEK-Basis (Polyetheretherketon), ein auf dem Markt erhältlicher Kationentauscher, Typ IR120, der Firma Rohm und Haas.
Das Harz IR120 ist nicht identisch, aber vergleichbar mit dem HCR-S Typ der Firma Dow Chemicals, dem S100 Typ der Firma Bayer, dem CF Typ der Firma Mitsubishi Chemicals und dem C100 Typ der Firma Purolite.
Ein quantitatives Maß für den Vorteil des erfindungsgemäßen Verfahrens ist der Erhalt der katalytischen Aktivität bei den untersuchten extremen Bedingungen. Als Maß dafür kann die Austauschkapazität des Kationenaustauschers genommen werden, da diese grundsätzlich für die katalytische Wirkung verantwortlich ist. Deswegen wurden die Austauschkapazitäten der Harze jeweils vor und nach der Veresterungsreaktion gemessen. Hierbei wurde zuvor das Harz im Vakuumtrockenschrank bei 80°C getrocknet und gewogen, sodaß sich die folgenden Kapazitätsangaben auf die Trockenmasse beziehen. Die Kapazität selbst wird als Austauschkapazität von Protonen gegen Natriumionen gemessen. Nach einer Verweilzeit von einer Stunde im Rührkessel konnten folgende Kapazitäten der Harze gemessen werden:
Hochtemperaturbeständiger Kationenaustauscher auf PEEKK-Basis: Vor der Veresterung 1,232 mMol SO₃H/g, nach der Veresterung 0,897 mMol SO₃H/g (entsprechend 73 % der ursprünglichen Kapazität).
Hochtemperaturbeständiger Kationenaustauscher auf PEEK-Basis: Vor der Veresterung 1,169 mMol SO₃H/g, nach der Veresterung 0,793 mMol SO₃H/g (entsprechend 68 % der ursprünglichen Kapazität).

Kationenaustauscher IR120: vor der Veresterung 3,440 mMol SO₃H/g, nach der Veresterung: 0,03 mMol SO₃H/g (entsprechend 1 % der ursprünglichen Kapazität)
In den Diagrammen 1 und 2 werden die Ergebnisse in Form von Balkendiagrammen dargestellt.

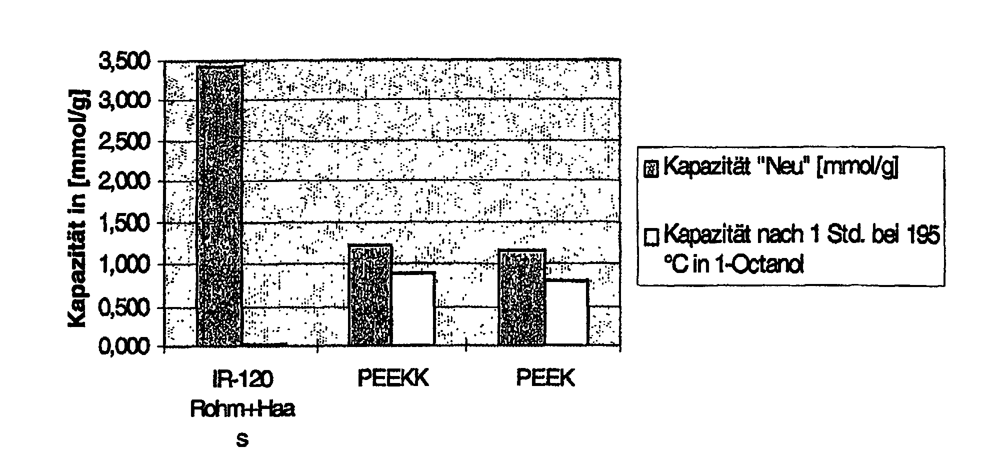

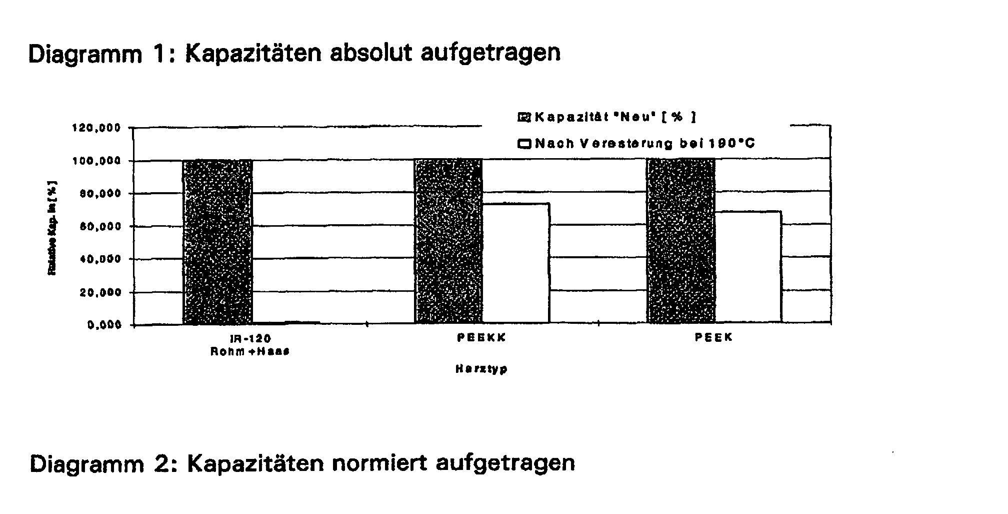

## Patentansprüche

1. Verwendung von sulfonierten aromatischen Polyetherketon-Kationenaustauschern als katalytisch aktive Materialien.

2. Verwendung nach Anspruche 1, **dadurch gekennzeichnet, daß** die sulfonierten aromatischen Polyetherketon-Kationenaustauscher in der heterogenen und/oder sauren Katalyse verwendet werden.

3. Verwendung nach Anspruch 1 oder 2, **dadurch gekennzeichnet, daß** die Verwendung bei Temperaturen aus dem Bereich von 20 - 300, bevorzugt 150 - 280, besonders bevorzugt von 160 - 250 °C stattfindet.

## Claims

1. Use of sulfonated aromatic polyether ketone cation exchangers as catalytically active materials.

2. Use according to Claim 1, **characterized in that** the sulfonated aromatic polyether ketone cation exchangers are used in heterogeneous and/or acid catalysis.

3. Use according to Claim 1 or 2, **characterized in that** the use takes place at temperatures from the range 20-300, preferably 150-280, particularly preferably 160 - 250, °C.

## Revendications

1. Utilisation d'échangeurs cationiques à base de polyéthercétones aromatiques sulfonées comme matières actives catalytiquement.

2. Utilisation suivant la revendication 1, **caractérisée en ce que** les échangeurs cationiques à base de polyéthercétones aromatiques sulfonées sont utilisés en catalyse hétérogène et/ou en catalyse acide.

3. Utilisation suivant la revendication 1 ou 2, **caractérisée en ce que** l'utilisation a lieu à des températures de l'ordre de 20 à 300, de préférence de 150 à 280 et tout particulièrement de 160 à 250°C.
